# EUROPÄISCHE PATENTANMELDUNG

(11) **EP 4 293 613 A1**
(43) Veröffentlichungstag der Anmeldung: **20.12.2023**
(21) Anmeldenummer: 22179768.1
(22) Anmeldetag: 19.06.2022
(51) Int. Cl.: G06T 7/00, G06T 7/11

(54) **IDENTIFIZIEREN UND CHARAKTERISIEREN VON ZYSTISCHEN LÄSIONEN DER BAUCHSPEICHELDRÜSE**

(71) Anmelder: Bayer AG, 51373 Leverkusen (DE)
(72) Erfinder: Die Erfindernennung liegt noch nicht vor
(74) Vertreter: BIP Patents

(57) **Zusammenfassung**

Die vorliegende Erfindung befasst sich mit dem technischen Gebiet der Identifizierung und Charakterisierung von zystischen Läsionen der Bauchspeicheldrüse. Gegenstände der vorliegenden Erfindung sind ein computer-implementiertes Verfahren, eine Vorrichtung und ein computerlesbares Speichermedium zur Identifizierung und Charakterisierung von zystischen Läsionen der Bauchspeicheldrüse.

## Beschreibung

Die vorliegende Erfindung befasst sich mit dem technischen Gebiet der Identifizierung und Charakterisierung von zystischen Läsionen der Bauchspeicheldrüse. Gegenstände der vorliegenden Erfindung sind ein computer-implementiertes Verfahren, eine Vorrichtung und ein computerlesbares Speichermedium zur Identifizierung und Charakterisierung von zystischen Läsionen der Bauchspeicheldrüse.

Die Bauchspeicheldrüse, auch als das Pankreas bezeichnet, ist ein im Bauchraum zwischen Leber, Milz, Magen und Zwölffingerdarm liegendes Drüsenorgan.

Die Bauchspeicheldrüse ist zugleich eine exokrine und endokrine Drüse; als exokrine Drüse produziert sie Verdauungsenzyme, als endokrine Drüse Hormone (wie z.B. Insulin und Glucagon).

Der von der Bauchspeicheldrüse gebildete Bauchspeichel ist ein wasserklares Sekret, das Enzyme für die Verdauung der Nahrung enthält. Der Bauchspeichel sammelt sich zunächst in kleinen Seitengängen, die dann, einem Kanalsystem ähnlich, in den Pankreas-Hauptgang münden. Der Pankreas-Hauptgang mündet an der sogenannten Papille in den Zwölffingerdarm (Duodenum).

In der Bauchspeicheldrüse können Zysten auftreten. Im Vergleich zu anderen Organen sind Zysten in der Bauchspeicheldrüse eher selten. Sie verursachen oft keine Beschwerden und werden häufig zufällig entdeckt. Man geht von einer Häufigkeit (Prävalenz) in der Bevölkerung von ca. 2,5% aus.

Es gibt mehr als 15 unterschiedliche Zysten-Arten im Pankreas und die meisten dieser Zysten sind harmlos. Einige Zysten in der Bauchspeicheldrüse können jedoch Vorstufen eines bösartigen Tumors darstellen und zu einem Pankreaskarzinom entarten.

Zystische Läsionen der Bauchspeicheldrüse werden dank des weit verbreiteten Einsatzes von hochauflösenden abdominalen Bildgebungsverfahren zunehmend erkannt. Mittels Sonografie des Bauchraumes lassen sich bereits viele zystische Veränderungen erkennen. Ergänzt wird die Diagnostik durch moderne Schnittbildverfahren wie Computertomographie (CT) und Magnetresonanztomografie.

Aufgabe der vorliegenden Erfindung war es, Radiologen bei der Identifizierung und Charakterisierung von zystischen Läsionen der Bauchspeicheldrüse zu unterstützen.

Diese Aufgabe wird durch die Gegenstände der unabhängigen Patentansprüche gelöst. Bevorzugte Ausführungsformen finden sich in den abhängigen Patentansprüchen, in der vorliegenden Beschreibung sowie in den Zeichnungen.

Ein erster Gegenstand der vorliegenden Erfindung ist ein computerimplementiertes Verfahren umfassend die Schritte:
- Empfangen mindestens einer radiologischen Aufnahme, wobei die mindestens eine radiologische Aufnahme eine Bauchspeicheldrüse eines Untersuchungsobjekts zeigt,
- Segmentieren der Bauchspeicheldrüse aus der mindestens einen radiologischen Aufnahme,
- Segmentieren von Teilen der Bauchspeicheldrüse aus der mindestens einen radiologischen Aufnahme,
- Identifizieren einer oder mehrerer Zysten in den identifizierten Teilen der Bauchspeicheldrüse,
- Klassifizieren der einen oder der mehreren Zysten,
- Ermitteln einer oder mehrerer Maßzahlen für die einen oder die mehreren Zysten,
- Ausgeben mindestens einer Repräsentation der Bauchspeicheldrüse, wobei in der mindestens einen Repräsentation die einen oder mehreren Zysten gekennzeichnet sind.

Ein weiterer Gegenstand der vorliegenden Erfindung ist eine Vorrichtung, umfassend einen Prozessor und einen Arbeitsspeicher, in dem ein Computerprogramm geladen werden kann, wobei das Computerprogramm den Prozessor veranlasst, die folgenden Schritte auszuführen:
- Laden mindestens einer radiologischen Aufnahme in den Arbeitsspeicher, wobei die mindestens eine radiologische Aufnahme eine Bauchspeicheldrüse eines Untersuchungsobjekts zeigt,
- Segmentieren der Bauchspeicheldrüse aus der mindestens einen radiologischen Aufnahme,
- Segmentieren von Teilen der Bauchspeicheldrüse in der mindestens einen radiologischen Aufnahme,
- Identifizieren einer oder mehrerer Zysten in den identifizierten Teilen der Bauchspeicheldrüse,
- Klassifizieren der einen oder der mehreren Zysten,
- Ermitteln einer oder mehrerer Maßzahlen für die einen oder die mehreren Zysten,
- Ausgeben mindestens einer Repräsentation der Bauchspeicheldrüse, wobei in der mindestens einen Repräsentation die einen oder mehreren Zysten gekennzeichnet sind.

Ein weiterer Gegenstand der vorliegenden Erfindung ist ein computerlesbares Speichermedium, umfassend Befehle, die bei einer Ausführung durch einen Computer diesen veranlassen, die folgenden Schritte auszuführen:
- Laden mindestens einer radiologischen Aufnahme in den Arbeitsspeicher, wobei die mindestens eine radiologische Aufnahme eine Bauchspeicheldrüse eines Untersuchungsobjekts zeigt,
- Segmentieren der Bauchspeicheldrüse aus der mindestens einen radiologischen Aufnahme,
- Segmentieren von Teilen der Bauchspeicheldrüse aus der mindestens einen radiologischen Aufnahme,
- Identifizieren einer oder mehrerer Zysten in den identifizierten Teilen der Bauchspeicheldrüse,
- Klassifizieren der einen oder der mehreren Zysten,
- Ermitteln einer oder mehrerer Maßzahlen für die einen oder die mehreren Zysten,
- Ausgeben mindestens einer Repräsentation der Bauchspeicheldrüse, wobei in der mindestens einen Repräsentation die einen oder mehreren Zysten gekennzeichnet sind.

Die Erfindung wird nachstehend näher erläutert, ohne zwischen den Erfindungsgegenständen (Verfahren, Vorrichtung, Computerprogramm, computerlesbares Speichermedium) zu unterscheiden. Die nachfolgenden Erläuterungen sollen vielmehr für alle Erfindungsgegenstände in analoger Weise gelten, unabhängig davon, in welchem Kontext (Verfahren, Vorrichtung, Computerprogramm, computerlesbares Speichermedium) sie erfolgen.

Wenn in der vorliegenden Beschreibung oder in den Patentansprüchen Schritte in einer Reihenfolge genannt sind, bedeutet dies nicht zwingend, dass die Erfindung auf die genannte Reihenfolge beschränkt ist. Vielmehr ist denkbar, dass die Schritte auch in einer anderen Reihenfolge oder auch parallel zueinander ausgeführt werden; es sei denn, ein Schritt baut auf einem anderen Schritt auf, was zwingend erforderlich macht, dass der aufbauende Schritt nachfolgend ausgeführt wird (was im Einzelfall aber deutlich wird). Die genannten Reihenfolgen stellen damit bevorzugte Ausführungsformen der Erfindung dar.

Die Erfindung wird an einigen Stellen in Bezug auf Zeichnungen näher erläutert. Dabei sind in den Zeichnungen konkrete Ausführungsformen mit konkreten Merkmalen und Merkmalskombinationen dargestellt, die in erster Linie der Veranschaulichung dienen; die Erfindung soll nicht so verstanden werden, dass sie auf die in den Zeichnungen dargestellten Merkmale und Merkmalskombinationen beschränkt ist. Ferner sollen Aussagen, die bei der Beschreibung der Zeichnungen in Bezug auf Merkmale und Merkmalskombinationen getroffen werden, allgemein gelten, das heißt auch auf andere Ausführungsformen übertragbar und nicht auf die gezeigten Ausführungsformen beschränkt sein.

Die vorliegende Erfindung stellt Mittel bereit, die einen Radiologen bei der Identifizierung und Charakterisierung von zystischen Läsionen der Bauchspeicheldrüse unterstützen.

Als eine Läsion wird eine Schädigung, Verletzung oder Störung einer anatomischen Struktur oder physiologischen Funktion bezeichnet. In der medizinischen Bildgebung (Radiologie) werden Bezirke mit verändertem Signalverhalten als Läsionen zusammengefasst, insbesondere weil die Herkunft einer Läsion (Entzündung, Parasit, Tumor, Verletzung, Abnutzung und/oder andere) oftmals beim Erzeugen einer radiologischen Aufnahme, die die Läsion zeigt, noch nicht bekannt ist.

Eine Zyste ist ein häufig mit einer Flüssigkeit gefüllter Hohlraum im Gewebe eines Körpers.

Vorzugsweise werden eine oder mehrere der nachfolgend aufgeführten zystischen Läsionen identifiziert und charakterisiert: serös zystische Neoplasie (engl. *serous cystic neoplasm*, Abk.: SCN), muzinös zystische Neoplasie (engl. *mucinous cystic neoplasm*, Abk.: MCN), intraduktal papillär-muzinöse Neoplasie (engl.: *intraductal papillary mucinous neoplasm*, Abk.: IPMN), dysontogenetische Zysten (engl.: *dysontogenetic cysts*), Pseudozysten (engl. *pseudocysts*).

Die Identifizierung und Charakterisierung von zystischen Läsionen der Bauchspeicheldrüse erfolgt auf Basis einer oder mehrerer radiologischer Aufnahmen.

In einem ersten Schritt wird also mindestens eine radiologische Aufnahme empfangen, wobei die mindestens eine radiologische Aufnahme die Bauchspeicheldrüse eines Untersuchungsobjekts zeigt. Bei dem Untersuchungsobjekt handelt es sich um ein Wirbeltier, vorzugsweise ein Säugetier, besonders bevorzugt um einen Menschen.

Bei der mindestens einen radiologischen Aufnahme handelt es sich vorzugsweise um mindestens eine computertomografische Aufnahme (CT-Aufnahme), um mindestens eine magnetresonanztomografische Aufnahme (MRT-Aufnahme) und/oder um mindestens eine Ultraschallaufnahme.

Bei der mindestens einen radiologischen Aufnahme kann es sich um eine kontrastmittelfreie Aufnahme handeln, oder es kann sich um eine oder mehrere radiologische Aufnahmen vor und/oder nach der Verabreichung eines oder mehrerer Kontrastmittel handeln.

"Kontrastmittel" sind Substanzen oder Gemische von Substanzen, die die Darstellung von Strukturen und Funktionen des Körpers bei bildgebenden radiologischen Verfahren verbessern. Beispiele für Kontrastmittel sind in der Literatur zu finden (siehe z.B. A. S. L. Jascinth et al.: Contrast Agents in computed tomography: A Review, Journal of Applied Dental and Medical Sciences, 2016, Vol. 2, Issue 2, 143 - 149; H. Lusic et al.: X-ray-Computed Tomography Contrast Agents, Chem. Rev. 2013, 113, 3, 1641-1666; https://www.radiology.wisc.edu/wp-content/uploads/2017/10/contrast-agents-tutorial.pdf, M. R. Nough et al.: Radiographie and magnetic resonances contrast agents: Essentials and tips for safe practices, World J Radiol. 2017 Sep 28; 9(9): 339-349; L. C. Abonyi et al.: Intravascular Contrast Media in Radiography: Historical Development & Review ofRisk Factors for Adverse Reactions, South American Journal of Clinical Research, 2016, Vol. 3, Issue 1, 1-10; ACR Manual on Contrast Media, 2020, ISBN: 978-1-55903-012-0; A. Ignee et al.: Ultrasound contrast agents, Endosc Ultrasound. 2016 Nov-Dec; 5(6): 355-362).

Die mindestens eine radiologische Aufnahme zeigt die Bauchspeicheldrüse des Untersuchungsobjekts. Die mindestens eine radiologische Aufnahme kann eine zweidimensionale Repräsentation einer Schicht durch die Bauchspeicheldrüse sein oder eine solche Repräsentation umfassen. Vorzugsweise ist die mindestens eine radiologische Aufnahme eine dreidimensionale Repräsentation eines Untersuchungsbereichs des Untersuchungsobjekts, der die Bauchspeicheldrüse umfasst, oder ein Stapel an zweidimensionalen Repräsentationen von Schichten innerhalb des Untersuchungsbereichs, die, übereinandergestapelt, eine dreidimensionale Repräsentation bilden.

Üblicherweise liegen die radiologischen Aufnahmen in digitaler Form vor. Der Begriff "digital" bedeutet, dass die Repräsentationen von einer Maschine, in der Regel einem Computersystem, verarbeitet werden können. Unter "Verarbeitung" werden die bekannten Verfahren zur elektronischen Datenverarbeitung (EDV) verstanden. Ein Beispiel für ein übliches Format für eine radiologische Aufnahme ist das DICOM-Format (DICOM: *Digital Imaging and Communications in Medicine*) - ein offener Standard zur Speicherung und zum Austausch von Informationen im medizinischen Bilddatenmanagement.

In einer digitalen radiologischen Aufnahme werden Bildinhalte üblicherweise durch ganze Zahlen repräsentiert und gespeichert. In den meisten Fällen handelt es sich um zwei- oder dreidimensionale Bilder, die binär kodiert und gegebenenfalls komprimiert sein können. Bei der digitalen radiologischen Aufnahme kann es sich um eine Rastergrafik handeln, bei der die Bildinformation in einer gleichmäßigen Rasterung abgelegt ist. Rastergrafiken bestehen aus einer rasterförmigen Anordnung von so genannten Bildpunkten (Pixel) im Fall von zweidimensionalen Darstellungen oder Volumenelementen (Voxel) im Fall dreidimensionaler Darstellungen, denen jeweils eine Farbe bzw. eine Graustufe zugeordnet ist. Die Hauptmerkmale einer 2D-Rastergrafik sind daher die Bildgröße (Breite und Höhe und ggf. Tiefe gemessen in Pixeln/Voxeln, umgangssprachlich auch Bildauflösung genannt) sowie die Farbtiefe. Jedem Bildpunkt/Volumenelement ist üblicherweise eine Farbe oder eine Graustufe zugeordnet. Die verwendete Kodierung der Farbe definiert sich unter anderem über den Farbraum und die Farbtiefe. Der einfachste Fall ist ein Binärbild, bei dem ein Bildpunkt/Volumenelement einen Schwarzweiß-Wert speichert. Bei einem Bild, dessen Farbe über den so genannten RGB-Farbraum definiert ist (RGB steht für die Grundfarben Rot, Grün und Blau), besteht jeder Bildpunkt / jedes Volumenelement aus drei Farbwerten, einem Farbwert für die Farbe Rot, einem Farbwert für die Farbe Grün und einem Farbwert für die Farbe Blau. Die Farbe eines Bildpunktes/Volumenelements ergibt sich durch die Überlagerung (additives Mischen) der drei Farbwerte. Der einzelne Farbwert ist z.B. in 256 unterscheidbare Stufen diskretisiert, die Tonwerte genannt werden und üblicherweise von 0 bis 255 reichen. Die Farbnuance "0" eines jeden Farbkanals ist üblicherweise die dunkelste. Haben alle drei Kanäle den Tonwert 0, erscheint der entsprechende Bildpunkt / das entsprechende Volumenelement schwarz; haben alle drei Kanäle den Tonwert 255, erscheint der entsprechende Bildpunkt / das entsprechende Volumenelement weiß. Bei der Ausführung der vorliegenden Erfindung werden digitale radiologische Aufnahmen bestimmten Operationen unterworfen. Die Operationen betreffen dabei überwiegend die Bildpunkte/Volumenelemente und/oder die Tonwerte der einzelnen Bildpunkte/Volumenelemente. Es gibt eine Vielzahl an möglichen digitalen Bildformaten, Farbkodierungen und Graustufenkodierungen. Vereinfachend wird in dieser Beschreibung davon ausgegangen, dass die vorliegenden radiologischen Aufnahmen Rastergrafiken mit einer spezifischen Zahl an Bildpunkten/Volumenelementen sind, die jeweils eine definierte Farbe bzw. Graustufe aufweisen. Diese Annahme soll jedoch in keiner Weise limitierend verstanden werden. Dem Fachmann der Bildbearbeitung ist klar, wie er die Lehre dieser Beschreibung auf radiologische Aufnahmen, die in anderen Bildformaten vorliegen, übertragen kann.

Die mindestens eine radiologische Aufnahme der Bauchspeicheldrüse kann von einem CT-Scanner, einem MRT-Scanner und/oder einem Ultraschallgerät empfangen werden und/oder aus einem oder mehreren Datenspeichern ausgelesen werden.

Die Identifizierung und/oder Charakterisierung von zystischen Läsionen der Bauchspeicheldrüse kann eine Reihe von Prozessen umfassen, die aufeinander aufbauen können. Diese Prozesse werden nachfolgend näher erläutert. Jeder Prozess dient üblicherweise einem Zeil. Für das Erreichen eines Zieles kann es mehrere Prozesse geben. Solche alternativen Prozesse werden nachfolgend ebenfalls erläutert. Vorzugsweise umfasst die vorliegende Erfindung einen oder mehrere der folgenden Prozesse: Segmentierung der Bauchspeicheldrüse, Segmentierung und/oder Identifizierung des Pankreas-Hauptgangs, Identifizierung und/oder Charakterisierung von Erweiterungen im Pankreas-Hauptgang, Identifizierung und/oder Charakterisierung und/ oder Lokalisierung von Läsionen in der Bauchspeicheldrüse, morphometrische Untersuchung der Bauchspeicheldrüse, Visualisierung der Bauchspeicheldrüse.

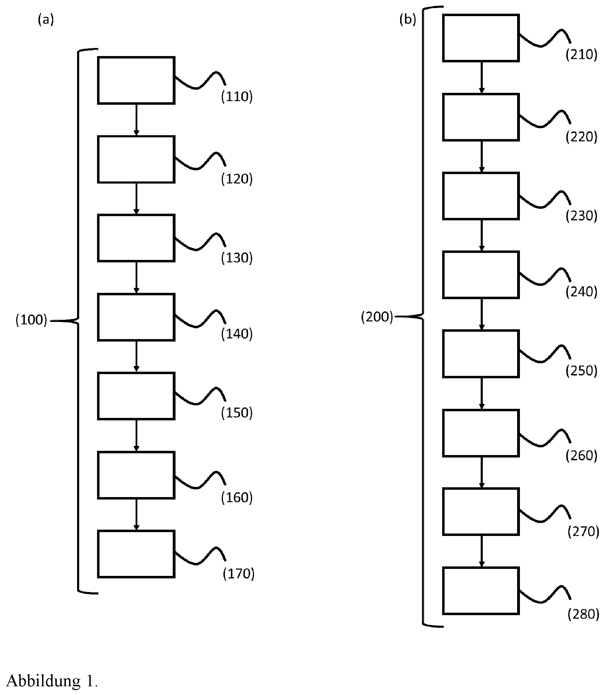

Abb. 1(a) zeigt beispielhaft und schematisch eine Ausführungsform des erfindungsgemäßen Verfahrens zum Identifizieren und Charakterisieren von zystischen Läsionen der Bauchspeicheldrüse. Dieses Verfahren (100) umfasst die Schritte:
(110) Empfangen mindestens einer radiologischen Aufnahme, wobei die mindestens eine radiologische Aufnahme eine Bauchspeicheldrüse eines Untersuchungsobjekts zeigt,
(120) Segmentieren der Bauchspeicheldrüse aus der mindestens einen radiologischen Aufnahme,
(130) Segmentieren von Teilen der Bauchspeicheldrüse aus der mindestens einen radiologischen Aufnahme,
(140) Identifizieren einer oder mehrerer Zysten in den identifizierten Teilen der Bauchspeicheldrüse,
(150) Klassifizieren der einen oder der mehreren Zysten,
(160) Ermitteln einer oder mehrerer Maßzahlen für die einen oder die mehreren Zysten,
(170) Ausgeben mindestens einer Repräsentation der Bauchspeicheldrüse, wobei in der mindestens einen Repräsentation die einen oder mehreren Zysten gekennzeichnet sind.

Abb. 1(b) zeigt beispielhaft und schematisch eine Ausführungsform des erfindungsgemäßen Verfahrens zum Identifizieren und Charakterisieren von zystischen Läsionen der Bauchspeicheldrüse. Dieses Verfahren (200) umfasst die Schritte:
(210) Empfangen mindestens einer radiologischen Aufnahme, wobei die mindestens eine radiologische Aufnahme eine Bauchspeicheldrüse eines Untersuchungsobjekts zeigt,
(220) Segmentieren der Bauchspeicheldrüse aus der mindestens einen radiologischen Aufnahme,
(230) Segmentieren von Teilen der Bauchspeicheldrüse aus der mindestens einen radiologischen Aufnahme,
(240) Identifizieren und Segmentieren des Pankreas-Hauptgangs
(250) Ermittlung der Zentrumslinie im Pankreas-Hauptgang
(250) Identifizieren einer oder mehrerer Zysten in den identifizierten Teilen der Bauchspeicheldrüse oder im Pankreas-Hauptgang,
(260) Klassifizieren der einen oder der mehreren Zysten,
(270) Ermitteln einer oder mehrerer Maßzahlen für die einen oder die mehreren Zysten,
(280) Ausgeben mindestens einer Repräsentation der Bauchspeicheldrüse, wobei in der mindestens einen Repräsentation die einen oder mehreren Zysten gekennzeichnet sind.

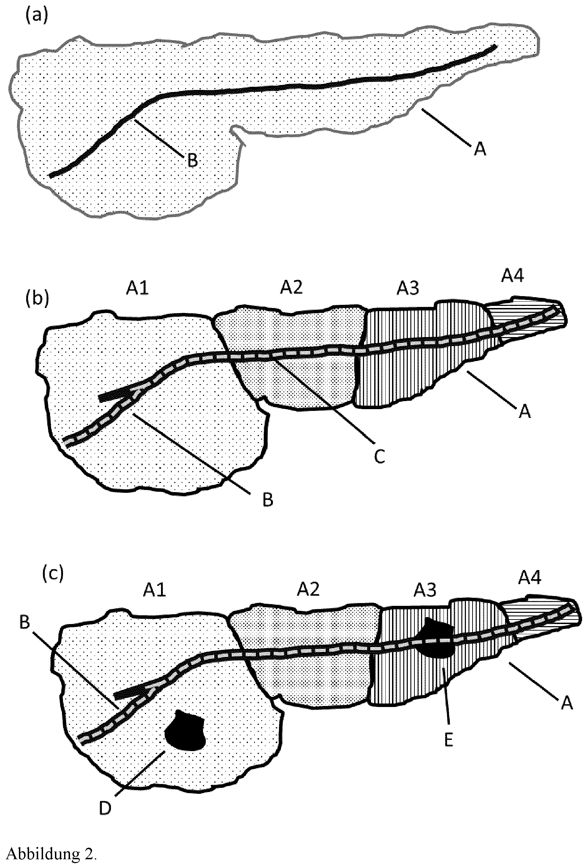

Abb. 2(a) zeigt eine schematische Darstellung der Bauspeicheldrüse (A) und des Pankreas-Hauptgangs (B)

Abb. 2(b) zeigt eine schematische Darstellung der Bauspeicheldrüse (A), die anhand der mindestens einen radiologischen Aufnahme in vier Segmenten (A1, A2, A3, A4) segmentiert wurde sowie den segmentierten Pankreas-Hauptgang (B) zur Ermittlung der Zentrumslinie (C).

Abb. 2(c) zeigt eine schematische Darstellung der Bauspeicheldrüse (A), die anhand der mindestens einen radiologischen Aufnahme in vier Segmenten (A1, A2, A3, A4) segmentiert wurde sowie eine zystische Läsion (D) in Segment A1 sowie eine weitere Zyste (E) entlang des segmentierten Pankreas-Hauptgangs (B) in Segment A3.

### Segmentierung der Bauchspeicheldrüse

Unter dem Begriff "Segmentieren" wird der Prozess der Aufteilung einer radiologischen Aufnahme in mehrere Segmente, die auch als Bildsegmente, Bildregionen oder Bildobjekte bezeichnet werden, verstanden. Die Segmentierung wird in der Regel verwendet, um Objekte und Grenzen (Linien, Kurven usw.) in radiologischen Aufnahmen zu lokalisieren. Aus einer segmentierten radiologischen Aufnahme können die lokalisierten Objekte vom Hintergrund getrennt, visuell hervorgehoben (z.B.: eingefärbt), gemessen, gezählt oder auf andere Weise quantifiziert werden.

Bei der Segmentierung wird jedem BildpunktNolumenelement einer radiologischen Aufnahme eine Kennzeichnung zugewiesen, so dass Bildpunkte/Volumenelemente mit derselben Kennzeichnung bestimmte Merkmale gemeinsam haben.

Im vorliegenden Fall werden in der mindestens einen radiologischen Aufnahme diejenigen Bildpunkte/Volumenelemente identifiziert und gekennzeichnet, die die Bauchspeicheldrüse repräsentieren; alle übrigen Bildpunkte/Volumenelemente repräsentieren dementsprechend andere Teile des Untersuchungsobjekts.

In einer bevorzugten Ausführungsform erfolgt die Segmentierung mit Hilfe eines Modells des maschinellen Lernens, das auf Basis von Trainingsdaten trainiert worden ist, jeden Bildpunkt / jedes Volumenelement einer radiologischen Aufnahme einer von mindestens zwei Klassen zuzuordnen, wobei mindestens eine Klasse Bildpunkte/Volumenelemente repräsentiert, die einen Teil der Bauchspeicheldrüse zeigen.

Ein "Modell des maschinellen Lernens" kann als eine computerimplementierte Datenverarbeitungsarchitektur verstanden werden. Das Modell kann Eingangsdaten empfangen und Ausgangsdaten auf der Grundlage dieser Eingangsdaten und Modell-Parametern liefern. Das Modell kann durch Training eine Beziehung zwischen den Eingabedaten und den Ausgabedaten erlernen. Beim Training können die Modell-Parameter angepasst werden, um eine gewünschte Ausgabe für eine bestimmte Eingabe zu liefern.

Beim Trainieren eines solchen Modells werden dem Modell Trainingsdaten präsentiert, aus denen es lernen kann. Das trainierte Modell des maschinellen Lernens ist das Ergebnis des Trainingsprozesses. Die Trainingsdaten umfassen neben Eingabedaten die korrekten Ausgabedaten (Zieldaten), die das Modell auf Basis der Eingabedaten erzeugen soll. Beim Trainieren werden Muster erkannt, die die Eingabedaten auf die Zieldaten abbilden.

Im Trainingsprozess werden die Eingabedaten der Trainingsdaten in das Modell eingegeben, und das Modell erzeugt Ausgabedaten. Die Ausgabedaten werden mit den Zieldaten verglichen. Modell-Parameter werden so verändert, dass die Abweichungen zwischen den Ausgabedaten und den Zieldaten auf ein (definiertes) Minimum reduziert werden.

Im Training kann eine Fehlerfunktion (engl.: *loss function*) verwendet werden, um die Vorhersagequalität des Modells zu bewerten. Die Fehlerfunktion kann so gewählt werden, dass sie eine erwünschte Beziehung zwischen Ausgabedaten und Zieldaten belohnt und/oder eine unerwünschte Beziehung zwischen Ausgabedaten und Zieldaten bestraft. Eine solche Beziehung kann z.B. eine Ähnlichkeit, eine Unähnlichkeit oder eine andere Beziehung sein.

Eine Fehlerfunktion kann verwendet werden, um einen Fehlerwert (engl.: *loss value*) für ein gegebenes Paar aus Ausgabedaten und Zieldaten zu berechnen. Das Ziel des Trainingsprozesses kann darin bestehen, die Parameter des maschinellen Lemmodells so zu verändern (anzupassen), dass der Fehlerwert für alle Paare des Trainingsdatensatzes auf ein (definiertes) Minimum reduziert wird.

Eine Fehlerfunktion kann z.B. die Abweichung zwischen den Ausgabedaten des Modells für bestimmte Eingabedaten und den Zieldaten quantifizieren. Handelt es sich bei den Ausgabedaten und den Zieldaten beispielsweise um Zahlen, kann die Fehlerfunktion die absolute Differenz zwischen diesen Zahlen sein. In diesem Fall kann ein hoher absoluter Wert der Fehlerfunktion bedeuten, dass ein oder mehrere Modell-Parameter in hohem Maße geändert werden müssen.

Bei Ausgabedaten in Form von Vektoren können beispielsweise Differenzmetriken zwischen Vektoren wie der mittlere quadratische Fehler, ein Kosinusabstand, eine Norm des Differenzvektors wie ein euklidischer Abstand, ein Tschebyscheff-Abstand, eine Lp-Norm eines Differenzvektors, eine gewichtete Norm oder jede andere Art von Differenzmetrik zweier Vektoren als Fehlerfunktion gewählt werden.

Bei höherdimensionalen Ausgaben, wie z.B. zweidimensionalen, dreidimensionalen oder höherdimensionalen Ausgaben, kann z.B. eine elementweise Differenzmetrik verwendet werden.

Alternativ oder zusätzlich können die Ausgabedaten vor der Berechnung eines Verlustwertes transformiert werden, z.B. in einen eindimensionalen Vektor.

Im vorliegenden Fall kann ein Modell des maschinellen Lernens trainiert werden, eine radiologische Aufnahme, die eine Bauchspeicheldrüse zeigt, als Eingabedaten entgegenzunehmen und, zumindest anteilig auf Basis der Eingabedaten und Modellparametern, Ausgabedaten erzeugen. Die Ausgabedaten können eine radiologische Aufnahme sein, in der jeder Bildpunkt / jedes Volumenelement, der/das einen Teil der Bauchspeicheldrüse repräsentiert, gekennzeichnet ist. Die Kennzeichnung kann beispielsweise eine Ziffer sein, die jedem Bildpunkt / jedem Volumenelement zugeordnet ist. Die Ziffer kann angeben, ob ein Bildpunkt / Volumenelement die Bauchspeicheldrüse repräsentiert oder einen anderen Teil des Untersuchungsobjekts repräsentiert. Zum Beispiel kann die Ziffer 1 angeben, dass ein Bildpunkt / Volumenelement einen Teil der Bauchspeicheldrüse repräsentiert und die Ziffer 0 angeben, dass ein Bildpunkt / Volumenelement keinen Teil der Bauchspeicheldrüse repräsentiert. Es ist auch möglich, dass eine Ziffer eine Wahrscheinlichkeit dafür angibt, dass ein Bildpunkt / Volumenelement einen Teil der Bauchspeicheldrüse repräsentiert.

Das Training des Modells des maschinellen Lernens kann auf Basis von Trainingsdaten erfolgen. Die Trainingsdaten können für eine Vielzahl von Untersuchungsobjekten unsegmentierte und segmentierte radiologische Aufnahmen der Bauchspeicheldrüse umfassen. Üblicherweise liegt für jedes Untersuchungsobjekt mindestens eine unsegmentierte radiologische Aufnahme und mindestens eine segmentierte radiologische Aufnahme vor. Die unsegmentierte radiologische Aufnahme wird dem Modell des maschinellen Lernens als Eingabedaten zugeführt. Die mindestens eine segmentierte radiologische Aufnahme dient als Zieldaten (auch als *ground truth* bezeichnet) und kann mit den Ausgabedaten, die vom Modell des maschinellen Lernens erzeugt werden, verglichen werden. Die segmentierten radiologischen Aufnahmen können von einem Experten (z.B. einem Radiologen) an einem Computer auf Basis der unsegmentierten radiologischen Aufnahmen erzeugt worden sein. Der Experte kann sich beispielsweise die unsegmentierten radiologischen Aufnahmen auf einem Bildschirm mit Hilfe eines Bildbearbeitungsprogramms anzeigen lassen und Bereiche in den unsegmentierten radiologischen Aufnahmen, die die Bauchspeicheldrüse repräsentieren, kennzeichnen. Die gekennzeichneten radiologischen Aufnahmen bilden die segmentierten radiologischen Aufnahmen.

Die Ausgabedaten können mit den segmentierten radiologischen Aufnahmen verglichen werden und die Abweichungen zwischen den Ausgabedaten und den segmentierten radiologischen Aufnahmen können mit Hilfe einer Fehlerfunktion quantifiziert werden. Modellparameter des Modells des maschinellen Lernens können modifiziert werden, um die Abweichungen zu reduzieren bzw. die Fehlerwerte auf ein (definiertes) Minimum zu reduzieren.

Bei dem Modell des maschinellen Lernens kann es sich beispielsweise um ein künstliches neuronales Netzwerk handeln, oder es kann ein solches umfassen.

Ein künstliches neuronales Netzwerk umfasst mindestens drei Schichten von Verarbeitungselementen: eine erste Schicht mit Eingangsneuronen (Knoten), eine n-te Schicht mit mindestens einem Ausgangsneuron (Knoten) und *n-2* innere Schichten, wobei *n* eine natürliche Zahl und größer als 2 ist.

Die Eingangsneuronen dienen zum Empfangen der radiologischen Aufnahmen. Üblicherweise gibt es ein Eingangsneuron für jeden Bildpunkt / jedes Volumenelement einer radiologischen Aufnahme. Es können zusätzliche Eingangsneuronen für zusätzliche Eingangswerte (z.B. Informationen zum Untersuchungsbereich, zum Untersuchungsobjekt und/oder zu Bedingungen, die bei der Erzeugung der radiologischen Aufnahme herrschten) vorhanden sein.

Die Ausgangsneuronen können dazu dienen, eine segmentierte Repräsentation auszugeben.

Die Verarbeitungselemente der Schichten zwischen den Eingangsneuronen und den Ausgangsneuronen sind in einem vorbestimmten Muster mit vorbestimmten Verbindungsgewichten miteinander verbunden.

Vorzugsweise handelt es sich bei dem künstlichen neuronalen Netz um ein so genanntes Convolutional Neural Network (kurz: CNN) oder es umfasst ein solches.

Ein CNN besteht üblicherweise im Wesentlichen aus Filtern (Convolutional Layer) und Aggregations-Schichten (Pooling Layer), die sich abwechselnd wiederholen, und am Ende aus einer Schicht oder mehreren Schichten von "normalen" vollständig verbundenen Neuronen (Dense / Fully Connected Layer).

Das Trainieren des neuronalen Netzes kann beispielsweise mittels eines Backpropagation-Verfahrens durchgeführt werden. Dabei wird für das Netz eine möglichst zuverlässige Vorhersage der segmentierten radiologischen Aufnahme angestrebt. Die Qualität der Vorhersage kann durch eine Fehlerfunktion beschrieben werden. Das Ziel ist die Minimierung der Fehlerfunktion. Das Einlernen eines künstlichen neuronalen Netzes erfolgt bei dem Backpropagation-Verfahren durch die Änderung der Verbindungsgewichte.

Im trainierten Zustand enthalten die Verbindungsgewichte zwischen den Verarbeitungselementen Informationen bezüglich der Segmentierung der Bauchspeicheldrüse, die verwendet werden kann, um, auf Basis einer neuen unsegmentierten radiologischen Aufnahme eine segmentierte radiologische Aufnahme zu erzeugen (vorherzusagen). Dabei bedeutet der Begriff "neu", dass die entsprechende radiologische Aufnahme nicht beim Training verwendet wurde.

Eine Kreuzvalidierungsmethode kann verwendet werden, um die Daten in Trainings- und Validierungsdatensätze aufzuteilen. Der Trainingsdatensatz wird beim Backpropagation-Training der Netzwerkgewichte verwendet. Der Validierungsdatensatz wird verwendet, um zu überprüfen, mit welcher Vorhersagegenauigkeit sich das trainierte Netzwerk auf unbekannte Daten anwenden lässt.

Das künstliche neuronale Netzwerk kann eine Autoencoder-Architektur aufweisen; z.B. kann das künstliche neuronale Netzwerk eine Architektur wie das U-Net aufweisen (siehe z.B. O. Ronneberger et al.: U-net: Convolutional networks for biomedical image segmentation, International Conference on Medical image computing and computer-assisted intervention, Seiten 234-241, Springer, 2015, https://doi.org/10.1007/978-3-319-24574-4_28).

Das künstliche neuronale Netzwerk kann ein *Generative Adversarial Network* (GAN) sein (siehe z.B. M.-Y. Liu et al.: Generative Adversarial Networks for Image and Video Synthesis: Algorithms and Applications, arXiv:2008.02793; J. Henry et al.: Pix2Pix GAN for Image-to-Image Translation, DOI: 10.13140/RG.2.2.32286.66887).

Das künstliche neuronale Netz kann ein Transfomer-Netzwerk sein (siehe z.B. D. Karimi et al.: Convolution-Free Medical Image Segmentation using Transformers, arXiv:2102.13645 [eess.IV]).

Das Modell des maschinellen Lernens, das zur Segmentierung der Bauchspeicheldrüse aus der mindestens einen radiologischen Aufnahme dient, wird in dieser Beschreibung auch als das erste Modell des maschinellen Lernens bezeichnet.

Eine andere Möglichkeit, die Bauchspeicheldrüse aus der mindestens einen radiologischen Aufnahme zu segmentieren, besteht in einer atlasbasierten Segmentierung. Bei der atlasbasierten Segmentierung wird Vorwissen ober das zu lokalisierende Objekt in Form eines Modells (dem Atlanten) verwendet. Das Vorwissen kann Wissen über die Form, die Ausrichtung, Kontinuität, Elastizität und/oder Glattheit des zu segmentierenden Objekts umfassen.

Bei einem atlasbasierten Verfahren kann ein zwei- oder dreidimensionales Modell der Bauchspeicheldrüse erzeugt werden. In einem solchen Modell, dem Atlanten, ist die Gestalt des zu segmentierenden Objekts vordefiniert. Der Atlas kann beispielsweise eine (manuell) segmentierte radiologische Aufnahme einer Durchschnitts-Bauspeicheldrüse sein. Möglichkeiten zur Erzeugung von Atlanten sind weiter hinten in der Beschreibung aufgeführt.

Bei der atlasbasierten Segmentierung sind für den Atlas diejenigen Bildpunkte / Volumenelemente, die auf die Bauchspeicheldrüse zurückzuführen sind (die einen Teil der Bauchspeicheldrüse repräsentieren), bekannt. Bei der atlasbasierten Segmentierung wird der Atlas einer oder mehreren Transformationen unterworfen, um ihn bestmöglich mit der mindestens einen radiologischen Aufnahme in Übereinstimmung zu bringen. Das Auffinden derjenigen Transformation(en), die den Atlas bestmöglich mit der mindestens einen radiologischen Aufnahme in Übereinstimmung bringt/bringen, wird auch als Registrierung bezeichnet. Ist die Registrierung abgeschlossen, können die übereinstimmenden Bildpunkte / Volumenelemente, die im Fall des Atlas die Bauchspeicheldrüse repräsentieren, auch im Fall der mindestens einen radiologischen Aufnahme als diejenigen Bildpunkte / Volumenelemente gekennzeichnet werden, die auf die Bauchspeicheldrüse zurückzuführen sind.

Man kann bei der Registrierung zwischen drei Transformationen unterscheiden: einer rigiden, einer affinen und einer elastischen Transformation. Eine rigide Transformation erlaubt Translationen und Rotationen des Atlanten, besitzt also sechs Freiheitsgrade im dreidimensionalen Raum. Eine affine Transformation erlaubt neben Translationen und Rotationen noch Skalierung und Scherung. Sie besitzt zwölf Freiheitsgrade im dreidimensionalen Raum. Unter dem Begriff elastische Transformation werden üblicherweise verschiedene Transformationsmodelle zusammengefasst, die unterschiedlich viele Freiheitsgrade besitzen können. Ein elastisches Transformationsmodell umfasst zum Beispiel ein Gitter, das über die mindestens eine radiologische Aufnahme gelegt werden kann und bei dem die einzelnen Gitterpunkte beweglich sind, sodass die mindestens eine radiologische Aufnahme entsprechend verzerrt werden kann. Dies erlaubt eine Anpassung des Atlas nicht nur in Bezug auf die Größe und Ausrichtung, sondern auch in Bezug auf die Form.

Da die Bauchspeicheldrüse zwischen Untersuchungsobjekten in ihrer Form variieren kann, und somit eine Rotation, Translation, Skalierung und/oder Scherung oftmals nicht ausreichen, um den Atlas bestmöglich auf die mindestens eine radiologische Aufnahme abzubilden, wird vorzugsweise ein elastisches Transformationsmodell verwendet.

Beispiele für elastische Transformationsmodelle sind BSpline-Registrierung, Finite-Elemente-Registrierung, Demons-Registrierung, Level-Set-Motion-Registrierung, Thin-Plate-Spline-Registrierung.

Die Registrierung ist üblicherweise ein iteratives Verfahren, bei dem nacheinander verschiedene Transformationen durchgeführt werden, und für jeden transformierten Atlanten geprüft wird, wie gut die Übereinstimmung zwischen dem transformierten Atlas und der mindestens einen radiologischen Aufnahme ist. Um zu überprüfen, wie gut der Atlas auf die mindestens eine radiologische Aufnahme passt, kann ein Ähnlichkeitsmaß (engl. *similarity measure*) bestimmt werden, das auf Helligkeitswerten der Bildpunkte / Volumenelement, ihrer räumlichen Verteilung, auf einer Histogrammauswertung und/oder auf anderen/weiteren Merkmalen basiert. Beispiele für Funktionen zur Berechnung eines Ähnlichkeitsmaßes sind *Sum of Squared Differences*, *Mutual Information.*

Die Durchführung von Transformationen und das Auffinden derjenigen Transformation(en), die für eine bestmögliche Übereinstimmung sorgt / sorgen, erfolgt üblicherweise nicht planlos, sondern es können bekannte Optimierungsverfahren verwendet werden wie beispielsweise gradientenbasierte Verfahren (z.B. Gradient Descent, Newton-Verfahren) oder nicht-gradientenbasierte Verfahren (z.B. Powell-Verfahren, Downhill-Simplex-Verfahren, Best-Neighbour-Verfahren, Goldener Schnitt).

Ein Atlas kann beispielsweise eine segmentierte radiologische Aufnahme einer Durchschnitts-Bauchspeicheldrüse sein, die beispielsweise durch Mittelung einer Vielzahl von radiologischen Aufnahmen einer Vielzahl von verschiedenen Untersuchungsobjekten gewonnen wird.

Ein solcher Atlas kann beispielsweise durch Registrierung und Überlagerung mehrerer radiologischer Aufnahmen mit einer Referenzaufnahme und Mittelung erzeugt werden. Die Referenzaufnahme kann dabei eine radiologische Aufnahme einer Bauchspeicheldrüse sein, deren Form, Größe und/oder Ausrichtung häufig beobachtet wird (Durchschnitts-Bauchspeicheldrüse).

Bevor die radiologischen Aufnahmen miteinander in Übereinstimmung gebracht werden, werden sie üblicherweise (manuell, z.B. durch einen Radiologen) segmentiert.

Die radiologischen Aufnahmen können nacheinander mit der Referenzaufnahme registriert werden und es kann nach jeder einzelnen Registrierung durch Mittelung der radiologischen Aufnahme mit der Referenzaufnahme eine neue Referenzaufnahme erzeugt werden, mit der dann die nächste radiologische Aufnahme registriert und fusioniert wird. Es ist aber auch denkbar, dass mehrere radiologische Aufnahmen zunächst mit der Referenzaufnahme registriert werden, bevor die radiologischen Aufnahmen zusammen mit der Referenzaufnahme zu einer neuen Referenzaufnahme (oder dem finalen Atlanten) gemittelt werden. Bei der Mittelung von zwei radiologischen Aufnahmen können beispielsweise die Grauwerte der Bildpunkte / Volumenelemente der einzelnen Aufnahmen jeweils durch zwei geteilt werden, um anschließend die durch zwei geteilten Grauwerte der beiden Aufnahmen zu addieren. Bei der Mittelung von *n* radiologischen Aufnahmen können die Grauwerte der Bildpunkte / Volumenelemente der einzelnen Aufnahmen jeweils durch die Zahl *n* geteilt werden, um anschließend die durch *n* geteilten Grauwerte aller Aufnahmen zu addieren, wobei *n* eine ganze Zahl ist.

Es können für Männer und Frauen unterschiedliche Atlanten verwendet werden. Ebenso können für Kinder und Erwachsene unterschiedliche Atlanten verwendet werden. Es können beispielsweise verschiedene Atlanten für verschiedene Altersstufen verwendet werden.

Weitere Details zur atlasbasierten Segmentierung können dem Stand der Technik entnommen werden (siehe z.B.: H. Park et al.: Construction of an Abdominal Probabilistic Atlas and its application in Segmentation, IEEE Transactions on medical imaging, 2003, Vol. 22, 483-492; P. Slagmolen et al.: Atlas based liver segmentation using nonrigid registration with a B-spline transformation model, MICCAI 2007, 197-206; O. Commowick et al.: Atlas-Based Delineation of Lymph Node Levels in Head and Neck Computed Tomography Images, In Radiotherapy Oncology, 2008, 87(2), 281-289; A. Shimizu et al.: Segmentation of multiple organs in non-contrast 3D abdominal CT images, International Journal of Computer Assisted Radiology and Surgery, 2007, Vol. 2, No.3-4, 35-142).

Eine andere Möglichkeit, die Bauchspeicheldrüse aus der mindestens einen radiologischen Aufnahme zu segmentieren, besteht in einer Verwendung eines verformbaren Modells. Der Hauptunterschied zwischen einer Atlasregistrierung und einem verformbaren Modell liegt in der Formulierung des Problems. Verformbare Modelle werden üblicherweise als physische Körper mit einer definierten Elastizität und einer Kraft implementiert, die versucht, die Form der Körper beizubehalten, während die mindestens eine radiologische Aufnahme, die segmentiert werden soll, üblicherweise als potenzielles Feld mit einer Kraft dargestellt wird, die versucht, das Modell zu dehnen und/oder zu stauchen, um es anzupassen. Die bestmögliche Anpassung kann als eine Lösung mit minimaler Energie gefunden werden, bei der beide Kräfte im Gleichgewicht sind.

Ein verformbares Modell einer Bauchspeicheldrüse kann aus einer Vielzahl von segmentierten radiologischen Aufnahmen der Bauchspeicheldrüse einer Vielzahl von Untersuchungsobjekten gewonnen werden. Dabei kann aus der Vielzahl an segmentierten radiologischen Aufnahmen eine Durchschnitts-Bauchspeicheldrüse gewonnen werden. Ebenso kann die Variabilität der Bauchspeicheldrüse in Bezug auf Größe und/oder Form, die in der Vielzahl an segmentierten radiologischen Aufnahmen zu beobachten ist, aus den radiologischen Aufnahmen gewonnen werden. Diese Variabilität kann den Bereich erlaubter Verformungen definieren; d.h., Verformungen, die in der Realität nicht zu beobachten sind, können verboten sein oder mit einer vergleichsweise hohen Kraft verbunden sein, die angewandt werden muss, um die entsprechende Verformung zu realisieren. Für Verformungen, die häufig auftreten, ist der Kraftaufwand für ihre Realisierung vergleichsweise gering.

Weitere Details zur Segmentierung unter Verwendung eines verformbaren Modells können dem Stand der Technik entnommen werden (siehe z.B.: S.F.F. Gibson et al.: A Survey ofDeformable Modeling in Computer Graphics, Tech. Rep., 1997; T. McInemey et al.: Deformable models in medical image analysis: a survey, Medical Image Analysis, 1996, Vol. 1, No. 2, 91-108; C. Xu et al.: Image Segmentation Using Deformable Models, Handbook of Medical Imaging, SPIE Press, 2000, Vol. 2., 447-514; A. Konno et al.: A Hepato-Biliary-Pancreatic Deformable Model for a Simulation-Based Laparoscopic Surgery Navigation, 2020 IEEE/SICE International Symposium on System Integration (SII), 2020, 39-44, doi: 10.1109/SII46433.2020.9025967; O. Ecabert et al.: Automatic Model-Based Segmentation of the Heart in CT Images, IEEE Transactions on Medical Imaging, Vol. 27, No. 9, 2008, 1189-1201).

In einem weiteren Schritt können Teile der Bauchspeicheldrüse aus der mindestens einen (vorzugsweise segmentierten) radiologischen Aufnahme segmentiert werden. Bei den Teilen handelt es sich vorzugsweise um Teile der Bauchspeicheldrüse, wie sie bei jedem (gesunden) Untersuchungsobjekt vorliegen.

Die Bauchspeicheldrüse wird häufig in drei Teile unterteilt: einen Pankreas-Kopf, einen Pankreas-Körper und einen Pankreas-Schwanz, auch kurz als Kopf, Körper und Schwanz bezeichnet (siehe z.B. Fig. 4 in: T. Ohtsuka et al.: Modern Japanese Approach to Pancreatic Cancer, Pancreatic Cancer, 2016, Springer, https://doi.org/10.1007/978-1-4939-6631-8_49-2).

In einigen Publikationen wird eine Unterteilung in fünf Teile vorgenommen: Haken (*Processus uncinatus pancreatis*), Kopf, Hals, Körper und Schwanz (siehe z.B.: K.M. Ramonell et al.: Pancreas, Surgical Anatomy and Technique, Springer, 2021, https://doi.org/10.1007/978-3-030-51313-9_9).

Als ein weiteres Teil der Bauchspeicheldrüse kann der Pankreas-Hauptgang erachtet werden.

Vorzugsweise umfasst das Segmentieren von Teilen der Bauchspeicheldrüse in der mindestens einen radiologischen Aufnahme das Segmentieren eines oder mehrerer der nachfolgend aufgeführten Teile: Haken, Kopf, Hals, Körper, Schwanz, Pankreas-Hauptgang.

Vorzugsweise wird zumindest der Pankreas-Hauptgang aus der mindestens einen radiologischen Aufnahme segmentiert.

Ein oder mehrere der genannten Teile können, wie für das Segmentieren der Bauchspeicheldrüse beschrieben, mit Hilfe eines Modells des maschinellen Lernens aus der mindestens einen radiologischen Aufnahme segmentiert werden. Das Modell des maschinellen Lernens kann auf Basis von Trainingsdaten, in denen ein Teil oder mehrere Teile der Bauchspeicheldrüse (z.B. manuell durch einen Radiologen) segmentiert ist/sind, trainiert werden. Das Modell des maschinellen Lernens kann trainiert werden, die Segmentierung, die bei den Trainingsdaten beispielsweise manuell vorgenommen worden ist, auf neue radiologische Aufnahmen zu übertragen. Dabei ist es denkbar, dass für verschiedene Teile, die segmentiert werden sollen, verschiedene Modelle des maschinellen Lernens vorliegen, oder dass ein Modell des maschinellen Lernens trainiert wird, mehrere verschiedene Teile aus radiologischen Aufnahmen zu segmentieren. Das Modell des maschinellen Lernens, das zur Segmentierung der Bauchspeicheldrüse aus der mindestens einen radiologischen Aufnahme verwendet wird, kann beispielsweise trainiert sein, gleichzeitig die Bauchspeicheldrüse als Ganzes als auch ein oder mehrere Teile der Bauchspeicheldrüse aus der mindestens einen radiologischen Aufnahme zu segmentieren. Ferner kann das Modell des maschinellen Lernens trainiert sein, ein oder mehrere Teile der Bauchspeicheldrüse aus der ursprünglich empfangenen mindestens einen radiologischen Aufnahme zu segmentieren.

Ferner können ein oder mehrere Teile der Bauchspeicheldrüse durch ein atlasbasiertes Segmentierverfahren und/oder mit Hilfe eines verformbaren Modells aus der mindestens einen radiologischen Aufnahme segmentiert werden.

### Segmentieren und/oder Identifizieren des Pankreas-Hauptgangs

Ein Ziel der Identifizierung und/oder Charakterisierung zystischer Läsionen der Bauchspeicheldrüse kann die Identifizierung und/oder Charakterisierung von Zysten im Pankreas-Hauptgang sein. Um Läsionen im Pankreas-Hauptgang zu erkennen, kann in einem ersten Schritt der Pankreas-Hauptgang wie oben beschrieben in der mindestens einen radiologischen Aufnahme segmentiert werden.

In einer bevorzugten Ausführungsform wird eine Zentrumslinie (engl. *centerline*) für den aus der mindestens einen radiologischen Aufnahme segmentierten Pankreas-Hauptgang ermittelt. Eine Zentrumslinie ist eine imaginäre Linie, die entlang des Pankreas-Hauptgangs führt. Eine Zentrumslinie kann als eine Linie verstanden werden, auf die der Pankreas-Hauptgang zusammengeschrumpft werden kann. Eine Zentrumslinie zeigt den Verlauf des Pankreas-Hauptgang in Form einer eindimensionalen Kurve von einem Anfang zu einem Ende an, wobei die Informationen zur Ausdehnung des Pankreas-Hauptgangs in die beiden anderen Dimensionen auf Null reduziert ist.

Es gibt verschiedene Verfahren zur Ermittlung einer Zentrumslinie (siehe z.B.: M. Lenga et al.: Deep Learning Based Rib Centerline Extraction and Labeling, https://doi.org/10.1007/978-3-030-11166-3_9; A. Sironiet et al.: Multiscale Centerline Detection, IEEE Transactions on Pattern Analysis and Machine Intelligence, 2015, 38. 1-1. 10.1109/TPAMI.2015.2462363; US8229186B2).

Die vorliegende Erfindung ist auf kein spezifisches Verfahren festgelegt.

Die oben aufgeführten Verfahren zur Ermittlung einer Zentrumslinie setzen voraus, dass der Pankreas-Hauptgang aus der mindestens einen radiologischen Aufnahme segmentiert werden kann. In einigen radiologischen Aufnahmen ist der Pankreas-Hauptgang jedoch nicht oder nur anteilig zu erkennen, so dass eine Segmentierung insbesondere mit einem atlasbasierten Segmentierverfahren oder mit einem Segmentierverfahren auf der Basis eines verformbaren Modells Schwierigkeiten bereiten kann.

In einer bevorzugten Ausführungsform wird daher eine Zentrumslinie vorhergesagt. Dazu wird in einem ersten Schritt die Bauchspeicheldrüse aus der mindestens einen radiologischen Aufnahme, wie in dieser Beschreibung beschrieben, segmentiert. Dabei handelt es sich bei der mindestens einen radiologischen Aufnahme um eine dreidimensionale Aufnahme der Bauchspeicheldrüse oder um einen Stapel zweidimensionaler Aufnahmen der Bauchspeicheldrüse, aus dem eine dreidimensionale Repräsentation konstruiert werden kann. Verfahren zum Umwandeln von dreidimensionalen Darstellungen in einen Stapel zweidimensionaler Darstellungen und umgekehrt sind im Stand der Technik beschrieben (siehe z.B.: Aharchi M., Ait Kbir M.: A Review on 3D Reconstruction Techniques from 2D Images, DOI: 10.1007/978-3-030-37629-1_37; Ehlke, M.: 3D-Rekonstruktion anatomischer Strukturen aus 2D-Röntgenaufnahmen, DOI: 10.14279/depositonce-11553; https://neuro.debian.net/pkgs/nifti2dicom.html).

Bei der segmentierten Bauchspeicheldrüse handelt es sich also vorzugsweise um eine dreidimensionale Repräsentation. Optional können ein oder mehrere Teile des Bauchspeicheldrüse segmentiert sein. Aus der segmentierten mindestens einen radiologischen Aufnahme lassen sich die Volumenelemente (Voxel) bestimmen, die die Bauchspeicheldrüse nach außen begrenzen. Mit anderen Worten: aus der segmentierten mindestens einen radiologischen Aufnahme lässt sich eine Oberfläche der Bauchspeicheldrüse ableiten. Optional umfasst eine solche Oberfläche Informationen, zu welchem Teil der Bauchspeicheldrüse einzelne Volumenelemente gehören, d.h. welchen Teil der Bauchspeicheldrüse sie repräsentieren. In einem weiteren Schritt kann die Oberflächen-Repräsentation der Bauchspeicheldrüse einem Modell des maschinellen Lernens zugeführt werden. Das Modell des maschinellen Lernens kann anhand von Trainingsdaten trainiert sein, auf Basis der Oberflächen-Repräsentation eine Zentrumslinie vorherzusagen. Die Trainingsdaten können segmentierte radiologische Aufnahmen der Bauchspeicheldrüse einer Vielzahl von Untersuchungsobjekten umfassen, wobei in den segmentierten radiologischen Aufnahmen sowohl die Oberfläche der Bauchspeicheldrüse gekennzeichnet (segmentiert) ist als auch eine Zentrumslinie für den Pankreas-Hauptgang gekennzeichnet ist. Das Modell des maschinellen Lernens kann also trainiert sein, aus einer Oberflächen-Repräsentation einer Bauchspeicheldrüse eine Zentrumslinie vorherzusagen. Dabei können für das Trainieren des Modells des maschinellen Lernens segmentierte radiologische Aufnahmen derselben oder einer anderen Modalität als bei der Verwendung des trainierten Modells des maschinellen Lernens zur Vorhersage verwendet werden. Es ist also beispielsweise möglich, aus MRT-Aufnahmen segmentierte radiologische Aufnahmen für das Trainieren des Modells des maschinellen Lernens zu gewinnen, das Modell auf Basis der segmentierten MRT-Aufnahmen zu trainieren und das trainierte Modell zur Vorhersage einer Zentrumslinie auf Basis mindestens einer CT-Aufnahme zu verwenden. Es ist auch möglich, aus CT-Aufnahmen segmentierte radiologische Aufnahmen für das Trainieren des Modells des maschinellen Lernens zu gewinnen, das Modell auf Basis der segmentierten CT-Aufnahmen zu trainieren und das trainierte Modell zur Vorhersage einer Zentrumslinie auf Basis mindestens einer MRT-Aufhahme zu verwenden.

### Identifizieren und/oder Charakterisieren von Erweiterungen im Pankreas-Hauptgang

In einem weiteren Schritt können Erweiterungen (engl. *dilatations*) im Pankreas-Hauptgang identifiziert werden. Solche Erweiterungen können auf das Vorhandensein einer Zyste, einer Läsion, oder einer zystischen Läsion im entsprechenden Bereich hinweisen (siehe z.B. I. Karoumpalisa, D. K. Christodoulou: Cystic lesions of the pancreas, Ann Gastroenterol. 2016 Apr-Jun; 29(2): 155-161).

Zur Identifizierung von Erweiterungen entlang der Zentrumslinie des Pankreas-Hauptgangs können unterschiedliche Methoden angewendet werden. Die vorliegende Erfindung ist auf keine spezifische Methode festgelegt.

In einer Ausführungsform wird eine Ebene entlang der Zentrumslinie des Pankreas-Hauptgangs bewegt und es werden die Schnittbereiche der Ebene mit dem segmentierten Pankreas-Hauptgang ermittelt. Vorzugsweise verläuft die Ebene in jedem Punkt entlang der Zentrumslinie senkrecht zu der Zentrumslinie. Mit anderen Worten: zu vorzugsweise jedem Punkt der Zentrumslinie wird eine Tangente ermittelt und es wird eine Ebene um den Punkt herum aufgespannt, wobei die jeweilige Tangente die Flächennormale (auch als Normalvektor bezeichnet) zu der jeweiligen Ebene darstellt. Für jeden Punkt wird der Schnittbereich der jeweiligen Ebene mit dem segmentierten Pankreas-Hauptgang ermittelt.

Eine Zunahme des Schnittbereichs (eine Zunahme der Schnittfläche) kann auf das Vorhandensein einer Zyste oder einer Läsion oder einer zystischen Läsion im Pankreas-Hauptgang hinweisen. Es kann für jeden Punkt der Zentrumslinie die Größe des ermittelten Schnittbereichs mit einem vor-definierten Schwellenwert verglichen werden. Liegt die Größe des ermittelten Schnittbereichs oberhalb eines definierten Schwellenbereichs, so kann dies auf eine zystische Läsion hinweisen. Schnittbereiche von Ebenen benachbarter Punkte der Zentrumslinie mit dem segmentierten Pankreas-Hauptgang können zu einem Volumenmodell des Pankreas-Hauptgangs zusammengefasst werden, aus dem das Volumen einer Erweiterung und die Ausdehnungen in die drei Raumrichtungen ermittelt werden können.

In einer weiteren Ausführungsform kann ein Modell des maschinellen Lernens, das als Regressionsmodell oder Klassifikationsmodell konfiguriert sein kann, verwendet werden, um Erweiterungen zu erkennen und/oder zu klassifizieren. Zum Beispiel kann ein Regressionsmodell trainiert werden, für Punkte entlang der Zentrumslinie den mittleren (z.B. arithmetisch gemittelten) Radius oder den mittleren (z.B. arithmetisch gemittelten) Durchmesser des Pankreas-Hauptgangs zu ermitteln. Es ist auch möglich, das Modell zu trainieren, den maximalen Radius oder den maximalen Durchmesser des Pankreas-Hauptgangs zu ermitteln. Das Regressionsmodell kann auch trainiert werden, die Größe der Schnittfläche des Pankreas-Hauptgangs mit einer Ebene senkrecht zur Tangente der Zentrumslinie an einer Reihe von Punkten der Zentrumslinie zu ermitteln. Ein Klassifikationsmodell kann trainiert werden, Schnittbilder, die durch den Pankreas-Hauptgang verlaufen, in eine von mindestens zwei Klassen einzuteilen, wobei eine Klasse beispielsweise Bilder von normalen (d.h. gesunden) Pankreas-Hauptgängen beinhaltet und mindestens eine weitere Klasse Bilder von erweiterten Pankreas-Hauptgängen erfasst, wobei die jeweilige Klasse die Ursache der Erweiterung angeben kann. Mit anderen Worten: das Klassifikationsmodell kann trainiert werden, Erweiterungen des Pankreas-Hauptgangs zu klassifizieren, wobei die Klasse die Art der Erweiterung und/oder die Ursache für die Erweiterung angeben kann.

Das Regressions- und/oder Klassifikationsmodell können anhand von Schnittbildern des Pankreas-Hauptgangs trainiert werden, die beispielsweise in sogenannter "Schiebefenster"- oder "Rollfenster"-Manier (engl. "sliding window" bzw. "rolling window", siehe z.B.: A. Helwan et al.: Sliding Window Based Machine Learning System for the Left Ventricle Localization in MR Cardiac Images, DOI: 10.1155/2017/3048181) entlang des Pankreas-Hauptgangs bewegt werden.

Erweiterungen des Pankreas-Hauptgangs können nach ihrer Detektion charakterisiert werden. Die Charakterisierung kann beispielsweise eine Klassifizierung sein, in der jede detektierte Erweiterung einer von mehreren Klassen zugeordnet wird, wobei die jeweilige Klasse angeben kann, um welche Art einer Erweiterung es sich handelt und/oder welche Ursache(n) die Erweiterung haben kann.

Ein dilatierter Pankreas-Hauptgang kann durch eine gutartige oder bösartige Erkrankung entstehen oder von soliden oder zystischen Pankreastumoren herrühren. Der Pankreas-Hauptgang kann auch aufgrund einer chronischen Pankreatitis oder infolge des Alterns oder normaler physiologischer Prozesse erweitert sein.

In einer Ausführungsform wird jede detektierte Erweiterung des Pankreas-Hauptgangs einer von mindestens zwei Klassen zugeordnet, wobei eine Klasse Erweiterungen beinhaltet, die auf intraduktal papillär-muzinöse Neoplasien zurückzuführen sind.

Die Klassifizierung von Erweiterungen kann beispielsweise mit Hilfe eines Modells des maschinellen Lernens erfolgen, das auf Basis von Trainingsdaten trainiert worden ist, mindestens eine Art von Erweiterung zu erkennen (z.B. intraduktal papillär-muzinöse Neoplasien) und mindestens eine radiologische Aufnahme, die eine solche Art der Erweiterung zeigt, einer Klasse mit dieser Art von Erweiterungen zuzuordnen.

In einer weiteren Ausführungsform wird ein auf maschinellem Lernen basierendes Modell verwendet, um Regionen der Zentrumslinie des Pankreas-Hauptgangs, die erweitert sein können, direkt in einer oder mehreren radiologischen Aufnahmen zu segmentieren und zu klassifizieren. Vorzugsweise operiert das Modell auf einem Bereich (*region of interest*), der den Pankreas-Hauptgang umfasst. Dieser Bereich kann automatisch ermittelt werden; es kann sich beispielsweise um einen Begrenzungsrahmen oder Begrenzungskasten (engl.: *bounding box*) handeln, der die segmentierte Bauchspeicheldrüse oder den segmentierten Pankreas-Hauptgang umschließt. Dieser Bereich kann aber auch von einem Nutzer festgelegt werden, beispielsweise indem ein Nutzer mittels einer grafischen Benutzeroberfläche einen Begrenzungsrahmen oder -kasten um den Bereich zeichnet. Das Modell des maschinellen Lernens kann konfiguriert sein, eine oder mehrere radiologische Aufnahmen oder einen oder mehrere Bereiche in einer oder mehreren radiologischen Aufnahmen zu segmentieren und dabei Erweiterungen des Pankreas-Hauptgangs zu markieren. Ein solches Modell des maschinellen Lernens kann beispielsweise auf einem Autoencoder basieren, der Eingabedaten (z.B. ein oder mehrere segmentierte oder unsegmentierte und/oder mit einer oder mehreren Begrenzungsrahmen oder -kasten versehene radiologische Aufnahmen) empfängt, eine komprimierte Repräsentation der Eingabedaten erzeugt und auf Basis der komprimierten Repräsentation eine oder mehrere segmentierte radiologische Aufnahmen erzeugt und ausgibt, in denen Erweiterungen in dem Pankreas-Hauptgang hervorgehoben und/oder markiert sind. Ein Beispiel für die Implementierung eines solchen Modells ist das so genannte U-Net (siehe z.B.: O. Ronneberger et al.: U-Net: Convolutional Networks for Biomedical Image Segmentation, https://doi.org/10.48550/arXiv.1505.04597). Das Modell des maschinellen Lernens kann ferner trainiert sein, (segmentierte) Erweiterungen zu klassifizieren.

Auch die Verwendung eines modellbasierten Ansatzes zur Identifizierung und/oder Charakterisierung von Erweiterungen des Pankreas-Hauptgangs, wie oben für die Segmentierung der Bauchspeicheldrüse beschrieben, kann verwendet werden.

### Identifizieren und/oder Charakterisieren und/oder Lokalisieren von Läsionen in der Bauchspeicheldrüse

Neben zystischen Läsionen innerhalb des Pankreas-Hautgangs können auch zystische Läsionen in anderen Bereichen der Bauchspeicheldrüse erkannt und charakterisiert werden.

In einem weiteren Schritt wird eine oder es werden mehrere Zysten, Läsionen oder zystische Läsionen in den segmentierten und identifizierten Teilen der Bauspeicheldrüse identifiziert. Zur Identifizierung möglicher Zysten in den identifizierten Teilen der Bauspeicheldrüse können unterschiedliche Methoden angewendet werden. Die vorliegende Erfindung ist auf keine spezifische Methode festgelegt.

In einer Ausführungsform können mögliche Zysten, Läsionen oder zystischen Läsionen in den segmentierten Bereichen der Bauchspeicheldrüse entsprechend ihrer Art und/oder ihrer Ursache klassifiziert werden. Das in der vorliegenden Erfindung bereitgestellte Verfahren ermöglicht die Identifizierung und/oder Klassifizierung verschiedener Arten von Zysten und/oder Läsionen, einschließlich zystischer Läsionen. Für eine Einordnung der Arten und Ursachen von Läsionen in der Bauchspeicheldrüse siehe z.B. "Ann Gastroenterol. 2016 April-Juni; 29(2): 155-161, doi: 10.20524/aog.2016.0007" und die darin zitierten Referenzen. Die Klassifikation der Läsionen kann erneut z.B. mit einem neuronalen Netzwerk oder einem statistischen Formmodell erfolgen.

In einer weiteren Ausführungsform können Zysten, Läsionen oder zystischen Läsionen der segmentierten und identifizierten Teile der Bauchspeicheldrüse unter Verwendung eines "Bounding-Box-Detektors" oder eines "minimalen bounding rectangle" identifiziert werden. Dies kann sowohl zweidimensional mit Hilfe von Slices als auch dreidimensional, d.h. volumetrisch erfolgen. In einer bevorzugten Ausführungsform kann die zweidimensionale Identifizierung von Läsionen über den YOLO-Ansatz zur Objekterkennung erfolgen. Diese Methode verwendet ein neuronales Netzwerk zur Vorhersage von Bounding-Boxen. In einer weiteren bevorzugten Ausführungsform kann die dreidimensionale Identifizierung von Läsionen unter Verwendung einer volumetrischen Bounding-Box-Detektor-Variante erfolgen.

In einer weiteren Ausführungsform können mögliche Läsionen in der Bauchspeicheldrüse klassifiziert werden, indem der entsprechende Schwerpunkt (COG) jeder Zyste oder Läsion mit einem Landmark- oder Schlüsselpunktdetektor bestimmt wird.

### Ermitteln einer oder mehrerer Maßzahlen

Die Identifizierung und die Klassifizierung möglicher Zysten, Läsionen oder zystischen Läsionen in der Bauchspeicheldrüse und/oder entlang der Zentrumslinie der Bauspeicheldrüse liefert wichtige Informationen über eine Reihe verschiedener Parameter, einschließlich, aber nicht beschränkt auf die Anzahl der Läsionen in der Bauchspeicheldrüse, ihre Lage innerhalb des Gewebes, einschließlich ihrer Lage in und innerhalb einer Pankreas-Subregion (z. B. Koordinaten, Größe), ihre Morphologie, d. h. ihrem maximalen und/oder mittleren (z.B. atithmetisch gemittelten Durchmesser und/oder einen Feret-Durchmesser der Läsionen (z.B. in mm), ihr Volumen (z.B. in mm³), ihre Kugelförmigkeit (siehe z.B. http://dx.doi.org/10.1680/jgeot.16.P.207) und/oder ihre Wechselwirkungen (z. B. ob sich die Läsionen überschneiden, überlappen oder in irgendeiner Weise miteinander verwandt sind).

Die genannten Maßzahlen und andere/weitere Maßzahlen können mit bekannten Methoden der Bildanalyse gewonnen werden (siehe z.B.: D. Luo: Pattern Recognition and Image Processing, Elsevier Science, 1998, ISBN: 9780857099761). Es gibt auch kommerziell erhältlich und frei verfügbare Software, die zur Bestimmung der genannten Merkmale und weiterer Merkmale eingesetzt werden kann. Als ein Beispiel sei die Software BioVoxxel (https://www.biovoxxel.de/) genannt.

### Ausgeben mindestens einer Repräsentation der Bauchspeicheldrüse

In einem weiteren Schritt wird mindestens einer Repräsentation der Bauchspeicheldrüse ausgegeben, wobei in der mindestens einen Repräsentation die einen oder mehreren identifizierten und charakterisierten Zysten, Läsionen oder zystischen Läsionen gekennzeichnet sind.

In einer Ausführungsform wird eine Repräsentation der Bauchspeicheldrüse bereitgestellt, wobei diese Repräsentation die Darstellung der in früheren Schritten und/oder Ausführungsformen erhaltenen Informationen über die Identifizierung und die Klassifizierung möglicher Zysten, Läsionen oder zystischer Läsionen in einem oder mehreren Segmenten der Bauchspeicheldrüse oder in der Zentrallinie der Bauchspeicheldrüse umfasst. Diese Repräsentation ermöglicht es dem Empfänger der Informationen, eine qualifizierte Beurteilung vorzunehmen und mögliche bedrohliche Zustände für die Gesundheit eines Subjekts zu diagnostizieren. Der Empfänger der Information ist vorzugsweise eine Person mit medizinischer Ausbildung, besonders bevorzugt eine Person mit fortgeschrittener medizinischer Ausbildung, besonders bevorzugt ein Techniker, besonders bevorzugt ein Arzt. Die Anzeige der Informationen kann Merkmale umfassen, die es dem Empfänger ermöglichen, mit den Informationen zu interagieren oder die Anzeige der Informationen zu verändern, d. h. indem er z. B. die Ansicht, den Maßstab, die Perspektive oder andere geeignete Parameter der Bilder ändert. In einer bevorzugten Ausführungsform kann der Empfänger der Information sein medizinisches Wissen nutzen, um die angezeigten Informationen zu verändern, z.B. um Parameter wie die Position der Zentrallinie der Bauchspeicheldrüse oder die Ränder einer möglichen Läsion, Zyste oder zystischen Läsion manuell zu korrigieren.

In einer bevorzugten Ausführungsform führt das erfindungsgemäße Computersystem Schritte, die in der vorliegenden Beschreibung beschrieben sind, nacheinander aus, und gibt nach jedem einzelnen Schritt oder einer Gruppe von Schritten eine Repräsentation der Bauchspeicheldrüse oder eines Bereichs des Körpers, der die Bauchspeicheldrüse ganz oder teilweise umfasst, gegenüber eine Nutzer aus, so dass der Nutzer das Ergebnis einzelner Schritte oder Gruppen von Schritten überprüfen und ggf. korrigierend eingreifen kann.

So kann das erfindungsgemäße Computersystem beispielsweise konfiguriert sein. Die segmentierte Bauchspeicheldrüse auszugeben. Der Nutzer kann prüfen, ob die Segmentierung korrekt ist, d.h. alle Bereiche in der mindestens einen radiologischen Aufnahme, die vom Computersystem der Bauchspeicheldrüse zugeordnet wurden, korrekt zugeordnet wurden. Hat das erfindungsgemäße Computersystem einen Bereich nicht korrekt zugeordnet, so kann das Computersystem konfiguriert sein, von dem Nutzer eine Eingabe z.B. mittels eine Computermaus entgegenzunehmen, in der der entsprechend nicht korrekt zugeordnete Bereich spezifiziert wird. Das erfindungsgemäße Computersystem kann konfiguriert sein, ein entsprechende Korrektur vorzunehmen, d.h. den Bereich wie vom Nutzer vorgegeben als zu der Bauchspeicheldrüse zugehörig bzw. nicht zugehörig zu markieren, bevor das erfindungsgemäße Computersystem mit nachfolgenden Schritten fortfährt.

Dies gilt analog auch für andere Segmentierungen, wie beispielsweise die Segmentierung der Bestandteile der Bauchspeicheldrüse, insbesondere des Pankreas-Hauptgangs, der Erwietrungen om Pankreas-Hauptgang und/oder identifizierten zystischen Läsionen.

## Patentansprüche

1. Verfahren zum Identifizieren und Charakterisieren von zystischen Läsionen der Bauchspeicheldrüse umfassend die Schritte:
- Empfangen mindestens einer radiologischen Aufnahme, wobei die mindestens eine radiologische Aufnahme eine Bauchspeicheldrüse eines Untersuchungsobjekts zeigt,
- Segmentieren der Bauchspeicheldrüse aus der mindestens einen radiologischen Aufnahme,
- Segmentieren von Teilen der Bauchspeicheldrüse aus der mindestens einen radiologischen Aufnahme,
- Identifizieren einer oder mehrerer Zysten in den identifizierten Teilen der Bauchspeicheldrüse,
- Klassifizieren der einen oder der mehreren Zysten,
- Ermitteln einer oder mehrerer Maßzahlen für die einen oder die mehreren Zysten,
- Ausgeben mindestens einer Repräsentation der Bauchspeicheldrüse, wobei in der mindestens einen Repräsentation die einen oder mehreren Zysten gekennzeichnet sind.

2. Vorrichtung, umfassend einen Prozessor und einen Arbeitsspeicher, in dem ein Computerprogramm geladen werden kann, wobei das Computerprogramm den Prozessor veranlasst, die folgenden Schritte auszuführen:
- Laden mindestens einer radiologischen Aufnahme in den Arbeitsspeicher, wobei die mindestens eine radiologische Aufnahme eine Bauchspeicheldrüse eines Untersuchungsobjekts zeigt,
- Segmentieren der Bauchspeicheldrüse aus der mindestens einen radiologischen Aufnahme,
- Segmentieren von Teilen der Bauchspeicheldrüse in der mindestens einen radiologischen Aufnahme,
- Identifizieren einer oder mehrerer Zysten in den identifizierten Teilen der Bauchspeicheldrüse,
- Klassifizieren der einen oder der mehreren Zysten,
- Ermitteln einer oder mehrerer Maßzahlen für die einen oder die mehreren Zysten,
- Ausgeben mindestens einer Repräsentation der Bauchspeicheldrüse, wobei in der mindestens einen Repräsentation die einen oder mehreren Zysten gekennzeichnet sind.

3. Computerlesbares Speichermedium, umfassend Befehle, die bei einer Ausführung durch einen Computer diesen veranlassen, die folgenden Schritte auszuführen:
- Laden mindestens einer radiologischen Aufnahme in den Arbeitsspeicher, wobei die mindestens eine radiologische Aufnahme eine Bauchspeicheldrüse eines Untersuchungsobjekts zeigt,
- Segmentieren der Bauchspeicheldrüse aus der mindestens einen radiologischen Aufnahme,
- Segmentieren von Teilen der Bauchspeicheldrüse aus der mindestens einen radiologischen Aufnahme,
- Identifizieren einer oder mehrerer Zysten in den identifizierten Teilen der Bauchspeicheldrüse,
- Klassifizieren der einen oder der mehreren Zysten,
- Ermitteln einer oder mehrerer Maßzahlen für die einen oder die mehreren Zysten,
- Ausgeben mindestens einer Repräsentation der Bauchspeicheldrüse, wobei in der mindestens einen Repräsentation die einen oder mehreren Zysten gekennzeichnet sind.

4. Verfahren, Vorrichtung oder computerlesbares Speichermedium nach einem der bevorstehenden Ansprüche, wobei das Segmentieren der Bauchspeicheldrüse aus der mindestens einen radiologischen Aufnahme oder das Segmentieren von Teilen der Bauchspeicheldrüse aus der mindestens einen radiologischen Aufnahme das Identifizieren und Segmentieren des Pankreas-Hauptgangs und/oder die Ermittlung der Zentrumslinie im Pankreas-Hauptgang umfasst.
